# FASCICULE DE BREVET EUROPEEN

(11) **EP 0 546 155 B1**
(45) Date de publication et mention de la délivrance du brevet: **26.05.1999**
(21) Numéro de dépôt: 92914635.5
(22) Date de dépôt: 01.07.1992
(51) Int. Cl.: A61B 17/435

(54) **PROCEDE DE FECONDATION EN CYCLE SPONTANE**
BEFRUCHTUNGSVERFAHREN BEI EINEM VON SELBST ABLAUFENDEN ZYKLUS
SPONTANEOUS-CYCLE FERTILIZATION METHOD

(30) Priorité: 01.07.1991 FR 9108177
(43) Date de publication de la demande: 16.06.1993
(73) Titulaire: BioFerTec, Ltd., Winchester, Massachusetts 01890 (US)
(72) Inventeur: Ranoux, Claude, Dr., Winchester, Massachusetts (US)
(74) Mandataire: CABINET BONNET-THIRION
(86) Numéro de dépôt international: FR9200615
(87) Numéro de publication internationale: WO9300863

(56) Documents cités:
- EP-A- 0 131 166
- EP-A- 0 153 190
- WO-A-88/08280
- FR-A- 2 614 899
- US-A- 4 010 738
- FERTILITY AND STERILITY vol. 52, no. 4, Octobre 1989, pages 617 - 621 FOULOT ET AL. 'In vitro fertilization without ovarian stimulation: a simplified protocol applied in 80 cycles'

## Description

Depuis la naissance de Louise BROWN en 1978, le premier enfant né de la fécondation in vitro, peu de modifications majeures sont survenues dans le processus de fécondation in vitro.

La stimulation ovarienne est maintenant classiquement utilisée en raison de la facilité à programmer la ponction des ovocytes. Les différents protocoles de stimulation ovarienne, citrate de clomiphène - hMG, hMG seul, FSH - hMG et plus récemment les agonistes de la LH-RH associés à l'hMG, ont augmenté de façon importante le nombre d'ovocytes recueillis lors de la ponction (de 2,5 à 3,5 en moyenne à 8 à 10 actuellement). Ce nombre plus important d'ovocytes recueillis a eu pour répercussion un nombre accru d'embryons. En raison du risque de grossesse multiple occasionné par le transfert de plus de trois embryons, la majorité des centres de procréation assistée se sont équipés d'appareil de congélation permettant de conserver les embryons et de les replacer secondairement. Ces protocoles de stimulation de par les quantités d'hormones utilisées et le nombre accru des examens (dosages hormonaux et examens échographiques) nécessaires à la surveillance de la croissance folliculaire, sont d'un coût très élevé.

La technique de fécondation proprement dite réalisée en laboratoire avec incubateur à CO2, hotte à flux laminaire, microscope inversé, nécessite un investissement initial important (voir DAMEWOOD, M.D.: In Vitro Fertilization : Insurance and financial considerations. Assisted Reproduction Review 1 : 38, 1991). A des temps de manipulation longs en raison de la complexité de la technique s'ajoute la nécessité de contrôler les paramètres tels le CO₂, la température et l'humidité relative dans l'incubateur. Ceci accroît d'autant le coût de la procédure. Ce coût, élevé, apprécié à $ 6 500 par tentative aux E.U.A et à environ 15 000 Frs en France, rend difficile l'extension et la diffusion de cette technique.

A cela s'ajoute des résultats, qui dans les meilleurs centres de fécondation, sont toujours inférieurs à la fécondité naturelle, soit 20 % de grossesse par cycle stimulé. Le coût élevé ainsi que les risques directement issus de ce type de traitement (hyperstimulation, grossesse multiple, stockage et manipulation d'embryons congelés) ont incité des administrations dans les pays dits développés à prendre des mesures afin de limiter l'extension des centres de procréation assistée. Ces mesures vont bien sûr à l'encontre des intérêts des couples inféconds.

L'expérience du demandeur en fécondation in vitro, démarrée en 1979, a permis de mettre au point une nouvelle procédure qui sera décrite ci-après.

### I. Culture Intra Vaginale (CIV).

Tout d'abord il est fait ici référence à la publication internationale WO 87/02879 déposée le 7 Novembre 1986 et publiée le 21 Mai 1987 et à la publication internationale WO 88/08280 déposée le 2 Mai 1988 publiée le 3 Novembre 1988.

Il a été observé tout d'abord que la technique de fécondation proprement dite pouvait être considérablement simplifiée. Ainsi il a été nous avons démontré par la technique dite "C I V E T E" (culture intra vaginale et transfert embryonnaire) que l'adjonction d'air enrichi de 5 % de CO₂ n'était pas nécessaire au maintien de bonnes conditions de culture. Mais afin d'éviter toute perturbation du milieu de culture il était nécessaire d'en remplir totalement le tube sans interposition d'air. De même afin de ne pas perturber de façon importante le pH du milieu de culture la concentration spermatique (responsable de la consommation métabolique) fut réduite de façon importante, 10 000 à 20 000 spermatozoïdes mobiles/ml alors qu'elle est d'au moins 50 000 spermatozoïdes mobiles/ml dans la technique classique. Cette technique nécessite moins de manipulations, aucun changement de milieu de culture.

Dans la technique classique de fécondation in vitro, une dénudation à l'aide d'une pipette ou d'aiguille est effectuée 18 à 30 heures après l'insémination. Cette procédure nécessite une certaine expertise et peut être traumatique pour l'embryon. Dans la technique de CIV, une dénudation spontanée des embryons survient dans plus de 80 % des cas. Cette dénudation spontanée correspond à un détachement spontané des cellules du cumulus qui entourent la corona (voir RANOUX C, AUBRIOT F.X., DUBUISSON J.B., CARDONE FOULOT H., POIROT C., CHEVALLIER O. : A new in vitro fertilization technique : intravaginal culture. Fertil Steril 49 : 654, 1988 et RANOUX, C. SEIBEL, M.C. : New techniques in fertilization : Intravaginal culture and microvolume straw. J In Vitro Fert "Embryo Transfer 7" : 6, 1990), elle est le reflet de la qualité de la culture et de celle des gamètes. En cas de toxicité, due soit aux moyens de stérilisation (éthylène oxyde), soit à la préparation des instruments lors de la ponction des ovocytes, une gangue de cellules denses d'aspect élastique entoure l'embryon ce qui nécessite de l'extraire afin d'en apprécier le stade de développement. L'embryon dans le cas d'une dénudation mécanique à l'aiguille ou à la pipette n'a jamais eu un stade de développement supérieur à 2 cellules, avec présence de fragments et d'un aspect irrégulier, asymétrique et anormal des cellules.

La technique de CIV est simple, peu coûteuse, elle ne requiert qu'un petit incubateur et un stéréomicroscope placé sous une hotte à flux laminaire de petite dimension. Elle ne nécessite aucune grosse infrastructure de laboratoire et notamment un incubateur à CO2. La technique de CIV implique une participation de la patiente d'où un meilleur contexte psychologique. Cette technique est plus physiologique, la fécondation étant réalisée dans le vagin à la température du corps avec les variations thermiques normalement observées en période périovulatoire. La fécondation réalisée en intravaginal, soulève moins de problèmes éthiques et présente moins de risque d'erreur entre les gamètes. De plus il n'existe pas de risque de panne d'incubateur pouvant compromettre la fécondation de plusieurs patientes. Cette technique par sa simplicité et sa standardisation ne nécessite pas une formation technique de haut niveau et peut être reproduite aisément sans modification des résultats.

Il a été par ailleurs démontré que ces résultats obtenus avec la technique de CIV étaient équivalents à ceux obtenus en fécondation in vitro classique, avec l'obtention de 15 % de naissance par ponction.

Dans un procédé de fécondation intra-utérine décrit dans le document WO A 88/08280 on remplit un conteneur avec du milieu de culture, au moins un ovocyte et des spermatozoïdes, on l'introduit dans la cavité utérine on le laisse pendant une durée déterminée pour obtenir la fécondation du ou des ovocytes, le contenu s'échappant ensuite dans la cavité utérine. Le prélèvement d'ovocytes est fait d'une manière classique et la patiente subit une stimulation ovarienne.

### II. Cycle naturel avec programmation de la ponction par injection d'hCG.

L'expérience de la stimulation ovarienne, basée sur plusieurs années, avec les différents protocoles évoqués plus haut, n'a pas permis d'observer de différence significative entre ces protocoles dans les pourcentages de naissance par tentative. Ces taux, même dans les meilleures équipes, se situent toujours entre 10 et 15 %. Ceci fut la principale des raisons qui incité à revenir au cycle naturel. Une autre de ces raisons furent les progrès réalisés depuis le premier "bébé éprouvette".

Lors des premières tentatives en cycle naturel, la croissance folliculaire était suivie par des dosages urinaires de l'estradiol (E 2) et de la LH sans contrôle échographique, d'où l'imprécision sur la maturité ovocytaire et sur le meilleur moment pour ponctionner l'ovocyte. Le suivi échographique associé à la survenue des dosages hormonaux sanguins rapides de l'estradiol et de la LH, ont permis l'obtention d'une plus grande précision dans la détermination de l'ovulation. L'utilisation d'hCG dans les cycles stimulés a permis de programmer la ponction des follicules 34 à 36 heures après l'injection évitant ainsi les ponctions ovocytaires à n'importe quelle heure du jour et de la nuit.

Enfin la survenue de nouveaux modes de ponction, non plus par coelioscopie, mais par contrôle échographique, plus récemment pratiqués par voie vaginale à l'aide d'une sonde vaginale, a permis une avancée technique importante. Cette ponction sous contrôle échographique ne nécessite en général pas d'anesthésie et permet de se dispenser d'une infrastructure chirurgicale. L'utilisation de ces progrès a permis de développer un protocole simplifié en cycle naturel avec programmation de la ponction par hCG (voir FOULOT H., RANOUX C., DUBUISSON JB., RAMBAUD AUBRIOT FX., POIROT C. : In vitro fertilization without ovarian stimulation : a simplified protocol applied in 80 cycles. Fertil Steril 52 : 617, 1989).

Le suivi de la croissance folliculaire ne commence qu'au 8 ou 9ème jour du cycle dépendant de la longueur normale du cycle de la patiente, le nombre de dosages hormonaux est très limité, en moyenne 5 par cycle avec seulement 2 ou 3 examens sonographiques.

Si la taille folliculaire est supérieure à 18 mm avec E2 supérieur à 180 pg/ml et sans pic de LH, l'hCG est injecté et l' ovocyte ponctionné 34 à 36 heures après. Si le diamètre est supérieur ou égal à 18 mm et E2 supérieur ou égal à 180 mg/ml avec la LH comprise entre 20 et 40 UI/l, l'hCG est immédiatement injecté et la ponction réalisée 24 heures après la réception des dosages sanguins. Enfin si la LH est supérieure à 40 UI/l, l'hCG n'est pas injecté et la ponction a lieu le lendemain du dosage.

Ce protocole simplifié a permis d'obtenir dans une étude portant sur 80 tentatives, un taux de grossesses de 22,5 % par cycle dont 17,5 % évolutives avec naissances d'enfants en bonne santé. Ce protocole offre de multiples avantages: le suivi de la croissance folliculaire est raccourci, simple et beaucoup moins coûteux. La ponction d'un seul follicule est rapide, facile et bien tolérée par la patiente. Cette technique ne nécessite pas d'anesthésie mais une simple prémédication. En raison de l'absence de médication, d'un temps technique réduit, d'une utilisation limitée d'instruments à usage unique, le coût de la procédure est considérablement réduit. L'absence de stimulation et l'obtention d'un seul ovocyte, ne représente aucun risque d'hyperstimulation. Les problèmes dus à la congélation embryonnaire sont éliminés. La possibilité de grossesse multiple avec le risque obstétrical et la morbidité néonatale qui lui sont inhérents, n'existe pas.

L'expérimentation a révélé des taux de croissance excessivement bas lorsque hCG est injecté après que le résultat du dosage sanguin montre un début de pic (doublement du taux de base avec LH < 40 UI/1) l'injection de hCG affectant la maturité du ou des ovocytes prélevés.

La présente invention a pour objet un procédé de fécondation in vitro adapté à être réalisé de façon ambulatoire dans lequel après ponction ovocytaire, on introduit un ou plusieurs de ces ovocytes dans un conteneur renfermant un milieu de culture, sans interposition de l'air ou CO₂, ainsi que des spermatozoïdes, avant l'introduction du conteneur dans la cavité vaginale ou l'utérus en vue de la fécondation du ou des ovocytes et la culture du ou des embryons, les valeurs de l'estradiol (E2) et de la LH étant surveillées par des dosages sanguins, caractérisé en ce que le procédé est réalisé sans stimulation du cycle ni injection de gonadotrophine chorionique avant ponction ovocytaire et la programmation de la ponction ovocytaire est basée sur les valeurs de l'E2 après doublement de la valeur de la LH pour la première fois.

Une injection de la hCG est effectuée immédiatement après la ponction ovocytaire.

De préférence, en présence d'une baisse de 30% ou d'une stabilité (c'est-à-dire + 10% de la valeur antérieure) de l'E2 par rapport à la valeur lors du doublement de la LH pour la première fois, la ponction est programmée 34 à 36 heures après le premier doublement de la valeur de la LH.

De préférence, en présence d'une augmentation de plus de 20% de l'E2 par rapport à la valeur du premier doublement de la valeur de la LH, la ponction est effectuée 30 à 34 heures après l'augmentation de plus de 20% de l'E2.

De préférence, la ponction ovocytaire est programmée aussitôt que trois dosages de E2 aient révélé une stabilité (c'est-à-dire + 10% de la valeur antérieure) après le premier doublement de la valeur de la LH.

De préférence l'ovocyte introduit dans le conteneur sans introduction d'air ou de CO₂ est maintenu à une température de 37° environ pendant deux à six heures.

Selon une possibilité importante, de la présente invention, le procédé peut être mis en oeuvre avec un seul ovocyte ponctionné et fécondé.

Pour mettre en oeuvre le procédé on prévoit un kit qui comprend un dispositif de dosage rapide de la LH, une aiguille de ponction d'ovocyte(s), une unité de filtration pour préparation de sperme, un conteneur pour fécondation des ovocytes et culture du ou des embryons avec ses moyens de contention noyés. On peut y ajouter des milieux de culture pour rinçage du follicule, préparation du sperme, maturation et fécondation de l'ovocyte. Chacun desdits éléments du kit est connu en soi.

Par ailleurs, le kit peut également comprendre un speculum jetable, un cathéter pour transfert du ou des embryons et/ou une seringue d'injection d'hCG.

Enfin, l'ensemble des éléments que comprend le kit est stérilisé, mis sous emballage hermétique et est à usage unique.

### III. La Fécondation In Vitro dans le Cabinet du spécialiste.

Devant la simplicité et les taux de succès obtenus dans chacune des procédures, une procédure totalement ambulatoire a été envisagée qui en permet la réalisation dans le cabinet d'un spécialiste en stérilité du couple. L'étude a comporté deux groupes :

GROUPE 1 (d'Août 1990 à Février 1991), 15 patientes furent incluses dans ce groupe, 11 de moins de 40 ans et 4 dont l'âge dépassait 40. 25 tentatives, par cette procédure de FIV dans l'office ont été effectuées. 3 patientes ont en effet bénéficié de 3 procédures, 4 de 2 procédures. Les indications pour lesquelles la FIV fut décidée, étaient pour 9 de ces patientes un facteur de stérilité tubaire, pour 3 d'entre elles un facteur de stérilité d'origine masculine, dans 1 cas une stérilité inexpliquée et enfin dans 2 cas un facteur cervical. Ce qui caractérise ce groupe 1 et qui le différencie fondamentalement du groupe 2, est la supplémentation de la phase lutéale. Dans ce groupe, cette supplémentation était de la progestérone synthétique injectée en intra musculaire chaque jour, à la dose de 25 ou 50 mg.

GROUPE 2 (de Mars 1991 à Mai 1991), 16 patientes furent incluses dans ce groupe. Chacune ayant bénéficié d'une tentative, soit 16 tentatives. 13 tentatives furent effectuées chez des patientes de moins de 40 ans, 3 chez des patientes de plus de 40 ans. Dans 9 cas la stérilité était d'origine tubaire, dans 4 d'origine inexpliquée, dans 2 une endométriose était responsable, dans le dernier cas un facteur masculin. Dans ce groupe la supplémentation de la phase lutéale fut effectuée à l'aide d'hCG comme initialement lors de notre étude sur cycle spontané. L'injection intra musculaire d'hCG fut réalisée le jour du transfert 1500 UI/l, et renouvelée 2 autres fois à trois jours d'intervalle.

Pour le protocole, la démarche fut pratiquement similaire dans les 2 groupes. Des différences sont survenues dans le groupe 2, sur lesquelles on reviendra, qui ont permis la mise au point d'un nouveau protocole en cycle spontané objet de cette demande.

Le suivi de la croissance folliculaire, les critères pour injecter l'hCG afin de programmer la ponction, furent les mêmes que ceux décrits précédemment excepté pour les 8 dernières tentatives. Le mode de ponction des follicules fut le même, sous contrôle échographique. Mais une meilleure expérience dans la technique a permis de réduire par moitié le temps moyen nécessaire à la procédure de 24 minutes dans le groupe 1 (8 à 50 minutes) à 12 minutes en moyenne dans le groupe 2 (7 à 25 minutes).

Dans la technique de CIV la procédure déjà décrite fut utilisée mise à part une modification sur laquelle on reviendra et en rapport avec le nouveau protocole du cycle spontané.

Un autre élément fut changé, le mode de contention du tube dans le vagin. Au départ effectué à l'aide d'un diaphragme, fut remplacé par une éponge plastique. En effet les diaphragmes aux Etats-Unis présentent sur leur surface une substance spermicide, qui aurait pu interférer sur la qualité de l'embryon. Le transfert embryonnaire fut effectué de façon classique à l'aide d'un cathéter.

### RESULTATS :

GROUPE 1. Sur les 25 tentatives, 18 conduirent à l'obtention d'ovocytes avec dans 4 tentatives l'obtention 2 ovocytes. Sur ces 22 ovocytes, 22 embryons furent obtenus après CIV, mais seulement 2 grossesses biochimiques résultèrent du transfert de ces embryons. Sur les 7 tentatives sans ovocytes, 5 le furent en raison d'un follicule déchiré ou rompu durant la ponction, 1 pour une ovulation avant ponction, dans la dernière l'ovocyte ne fut pas obtenu après plusieurs rinçages.

GROUPE 2. Sur les 16 tentatives, 16 ovocytes furent obtenus mais 2 d'entre eux présentaient une zone pellucide rompue, et donc seulement 14 embryons résultèrent de la fécondation en CIV. 4 grossesses furent obtenues dont 3 cliniques toujours évolutives avec battements cardiaques foetaux à l'échographie. Un autre résultat paraît être intéressant : c'est le pourcentage d'embryons spontanément dénudés dans le groupe 1 qui était de 36 % (8/22) alors que ce pourcentage atteignait 86 % dans le groupe 2 (12/14), avec des stades embryonnaires plus fréquemment à 4 ou 5 cellules dans ce groupe, alors qu'ils n'étaient le plus fréquemment qu'à 2 ou 3 cellules dans le groupe 1.

Ces résultats démontrent clairement une supériorité de la procédure utilisée dans le groupe 2, par rapport à celle du groupe 1. Cette différence est en partie due à une plus grande expérience dans la procédure, et à une supplémentation par l'hCG dans la phase luteale. On pense cependant que l'élément essentiel est le nouveau protocole en cycle spontané qui va être détaillée ci-après.

### NOUVEAU PROTOCOLE

En fait les 4 grossesses du groupe 2 ont été obtenues sur les 8 dernières tentatives en utilisant le nouveau protocole. Les 8 dernières tentatives ont été effectuées chez 8 patientes, 6 agées de moins de 40 ans et 2 de plus de 40. Les indications dans 4 cas étaient une stérilité d'origine tubaire, dans 2 d'origine inexpliquée, enfin, pour la dernière, d'origine masculine. Pour ces 8 tentatives l'hCG ne fut pas injecté et la programmation de la ponction fut décidée uniquement sur les valeurs de la LH en appréciant le moment du pic de LH. Les mêmes valeurs de la LH, que celles utilisées précédemment, furent retenues. L'obtention du premier doublement de la valeur de la LH de base avec un taux inférieur à 40 UI/l, était considéré comme le point de départ du pic. Ce doublement du taux de LH fut le plus souvent obtenu lors du prélèvement de sang du matin (entre 8 et 10 heures). La ponction était alors programmée 34 à 36 heures après le prélèvement de sang soit le lendemain dans la soirée. Un prélèvement de sang était alors systématiquement effectué dans l'après-midi (3 à 5 heures) révélant parfois une stagnation de la LH, avec une valeur de la LH à peu près identique à celle du matin et toujours inférieure à 40 UI/I. L'analyse de ces premiers résultats et l'absence de grossesse dans ce type de stagnation fait penser que la ponction a été réalisée trop tôt. Lorsque cette stagnation existe la ponction doit être différée au surlendemain matin pour une meilleure maturation ovocytaire.

Exception faite des patientes agées de plus de 40 ans, en raison du risque majeur d'ovulation avant ponction, la maturation ovocytaire semblant s'effectuer plus rapidement. La valeur de la LH du dosage suivant (en général le lendemain matin), révèle une valeur de la LH supérieure à 40 UI/l confirmant ainsi la stagnation du précédent dosage.

L'expérience du demandeur a montré que si le taux de E2 tombe (de 30% de la valeur antérieure) ou reste stable (à 10% au dessus ou en dessous de la valeur antérieure) par rapport aux taux de E2 lorsque la LH double pour la première fois sa valeur de base la ponction doit être programmée comme prévu 34 à 36 heures après le premier doublement de la LH. En fait la ponction sera programmée aussitôt que trois dosages de E2 donnent la même valeur après doublement de la valeur de base de la LH. Si le taux de E2 le lendemain du pic présumé de la LH a augmenté de plus de 20% au dessus du taux de doublement par rapport à la valeur de base, la ponction sera effectuée 30 à 34 heures après avoir atteint le taux supérieur de E2 (faux pic précoce de LH).

Une autre particularité a été d'injecter les 5000 UI d'hCG immédiatement après la ponction ovocytaire, la supplémentation de la phase lutéale par l'hCG s'effectuant comme précédemment décrit. Devant l'absence de précision sur la maturité de l'ovocyte, une phase de maturation fut ajoutée après le recueil ovocytaire. L'ovocyte est placé à 37°C durant 2 à 30 heures dans un tube entièrement rempli de milieu de culture (B2 de Menezo) sans interposition d'air et de CO2.

Les résultats révèlent la qualité de ce type de protocole. En effet sur les 4 patientes enceintes 3 sont des grossesses cliniques évolutives.
- 1 chez une patiente de plus de 40 ans, traitée pour endométriose.
- 1 chez une patiente de moins de 40 ans (39 ans 11 mois au moment de la procédure), traitée pour stérilité inexpliquée.
- 1 chez une patiente de 30 ans avec endométriose et facteur masculin pour laquelle cette technique avait été instituée dans un but diagnostic, afin de savoir si une fusion des 2 gamètes était possible. Cette patiente avait subi des inséminations par donneur avant cette tentative devant la sévérité du facteur masculin.

Enfin la grossesse avortive intéressait une patiente de 32 ans, traitée pour stérilité inexpliquée. La ponction semble avoir été effectuée trop tôt. L'analyse des 4 autres cas révèle :
- Pour l'une des patientes agées de 38 ans présentant des problèmes tubaires, un facteur masculin associé très sévère fut responsable de l'obtention d'un embryon à 2PN irréguliers, qui fut transféré bien que les chances de succès soient pratiquement inexistentes (la loi du Massachusetts oblige le transfert de tout embryon obtenu par procréation).
- Pour une autre patiente agée de 32 ans, un embryon de très belle qualité fut obtenu au stade de 6 cellules mais le transfert extrêmement difficile et traumatique (malgré l'injection de VALIUM, de VERSED et d'anesthésique local) est vraisemblablement responsable de l'insuccès.
- Pour la troisième patiente agée de 40 ans présentant un facteur tubaire, l'embryon obtenu n'était pas de belle qualité avec des blastomères asymétriques, irréguliers et la présence de corps résiduels.
- Enfin pour la dernière patiente agée de 35 ans présentant un facteur tubaire, un embryon à 3 cellules fut obtenu avec une ponction ovocytaire trop précoce.

### CONCLUSIONS

L'expérimentation préalable du cycle spontané avec programmation de la ponction par hCG, a révélé des taux de grossesses excessivement bas lorsqu'on injecte 5000 UI d'hCG à la réception des résultats, montrant un début du pic de LH (doublement du taux de base, avec LH < 40 UI/L). L'hypothèse serait que l'hCG est injecté à un moment inadéquat et de ce fait, interférerait sur la maturité de l'ovocyte. L'expérience du demandeur sur la ponction au pic de LH, où de fortes variations de taux de E2 allant de 120 à 350 pg/ml ont été observées à l'époque du pic de LH, a confirmé ce point. On a de même observé que le follicule était très fragile et se rompait fréquemment au moment de la ponction. Ces 2 constatations ont incité à ne plus injecter l'hCG avant la ponction pour bénéficier d'un cycle entièrement naturel.

Par contre l'injection des 5000 UI d'hCG immédiatement après la ponction paraît être fondamentale. En effet l'un des problèmes de la FIV, qui peut expliquer les faibles taux de succès obtenus, semble l'absence de synchronisme entre la croissance de l'embryon et la croissance de l'endomètre. L'embryon se situant dans des conditions de culture optimales se développerait peut être plus rapidement que naturellement. Le contraire serait observé pour l'endomètre dont la maturation dépend des sécrétions hormonales des cellules de la granulosa. Le potentiel des cellules de la granulosa peut être réduit au cours de la ponction en raison de cellules arrachées au cours de l'aspiration du liquide folliculaire et des différents rinçages du follicule. On a pensé à rétablir ce synchronisme par la conservation de l'embryon durant une brève période, 24 à 36 heures avant son transfert, à des températures en dessous de 37°C (American Fertility Society, 1990, P. 38), ou en conservant l'ovocyte à 4°C dans un milieu de culture à base de jaune d'oeuf et cryoprotecteur durant 24 heures avant l'insémination. En fait le mode le plus simple est peut être, comme on l'a fait, d'injecter de l'hCG juste après la ponction. L'hCG va ainsi stimuler les cellules de la granulosa et accroitre le taux sanguin de progestérone, ce qui aura pour effet de favoriser la maturation de l'endomètre aussi que sa réceptivité en vue d'une implantation embryonnaire. L'injection de hCG doit être retardée jusqu'au moment de l'insémination si la maturation de l'ovocyte excède 10 heures.

L'élément essentiel pour programmer la ponction parait être de suivre l'évolution de la LH sanguine. Programmer la ponction ovocytaire sur les résultats de 2 dosages sanguins journaliers de la LH peut paraître imprécis et peu efficace. Cependant les résultats obtenus sur les 8 premières tentatives en démontrent le contraire, tant par le nombre d'ovocytes recueillis (8/8), que par le nombre d'embryons obtenus (8/8). Ce nouveau protocole sur deux dosages sanguins de E2 le même jour apparaît donner des résultats équivalents supérieurs à ceux de la ponction sur injection de hCG..

L'élément essentiel réside dans l'obtention d'un dosage de LH au dessus de 40 UI/l. L'obtention d'un tel taux conduira à effectuer la ponction 24 heures après le prélèvement sanguin, et éventuellement à retarder le moment d'une ponction dont la programmation avait été décidée sur les premiers dosages de la LH. L'addition d'une phase de maturation ovocytaire à 37°C sans air enrichi en CO2, permet de parfaire la qualité ovocytaire.

La combinaison du cycle naturel avec ponction d'un seul ovocyte, sous contrôle échographique, et sa fertilisation en technique de CIV a permis pour la première fois la réalisation d'une fécondation in vitro dans le cabinet d'un spécialiste en infécondité. Cette technique entièrement réalisée en ambulatoire est simple, facilement reproductible, ne nécessite ni salle d'opération, ni anesthésie, ni moyen technique complexe. Les instruments utilisés sont simples, stéréomicroscopes, petite hotte à flux laminaire, incubateur à 37°C sans CO₂ de faible capacité. Tout cet équipement peut être facilement stocké dans un bureau pour une surface d'occupation au sol n'excédant pas 2,3 M2. Le petit matériel utilisé (aiguille à ponction, seringues, boîtes de pétri, tubes pour préparation du sperme, tube ou conteneur pour CIV avec son mode de contention noyés (éventuellement spéculum plastique, compresses, pinces plastiques, poche stérile pour couvrir la sonde vaginale) et milieu de culture pour rinçage du follicule, pour lavage du sperme, pour maturation et fécondation de l'ovocyte. Tous ces instruments étant stérilisés aux rayons gamma et emballés individuellement en poche cellophane) est un matériel à usage unique. La sélection de ce type d'équipement nécessite beaucoup d'attention et d'expérience, afin d'éviter une quelconque toxicité et d'obtenir l'équipement le plus adapté à ce type de procédure. C'est pourquoi nous avons pensé à réunir une sélection de ces meilleurs équipements sous le forme d'un kit en fécondation in vitro.

Au petit matériel précédemment cité pourrait venir s'ajouter l'unité de filtration pour préparation du sperme que j'ai développée et un dispositif de dosage rapide de la LH connu en soi, ainsi que les ampoules d'hCG utilisées pour supplémenter la phase lutéale. Au minimum le kit comportera donc un dispositif de dosage rapide de la LH, une aiguille de ponction d'ovocyte(s), un conteneur pour maturation des ovocytes selon la technique CIV et un conteneur pour fécondation des mêmes ovocytes ou éventuellement un conteneur pour la maturation et la fécondation des ovocytes. Selon la technique décrite dans la demande de brevet internationale WO 88/08280 déposée le 2 Mai 1988 le conteneur peut être au moins partiellement biodégradable.

Le kit représenterait un gain de temps et d'énergie considérable pour le praticien utilisant ce type de procédure, il serait la garantie d'une procédure réalisée dans les meilleures conditions.

Le coût de cette procédure est considérablement réduit, rendant ainsi la FIV accessible à plus de couples inféconds. L'association du cycle naturel et de la CIV est beaucoup plus physiologique et ne présente pas les mêmes problèmes éthiques que la technique conventionnelle. Les avantages précédemment énumérés de cette procédure associés à des résultats, certes préliminaires, mais très satisfaisants, en font une technique de choix dans le traitement de la stérilité. Cette procédure ne sera plus uniquement utilisée comme le dernier recours pour un couple infécond mais comme le premier examen à pratiquer chez ce couple afin d'apprécier avec certitude, le pouvoir fécondant des gamètes. Ceci permettra d'éviter des bilans d'infécondité longs et très coûteux pour tous les couples. Ces bilans ne seront alors effectués que pour les couples présentant une absence de fusion des gamètes au cours de la procédure permettant ainsi de consacrer plus d'argent aux réels problèmes de stérilité.

Ce cycle naturel pourra être combiné aussi avec la technique de fécondation en paillette que nous avons déjà développée. Une simplification extrême de la FIV surviendra avec une paillette biodégradable. La procédure ne nécessitera alors pas de transfert embryonnaire et la seule intervention technique humaine aura pour but de préparer et associer les 2 gamètes (ovocyte et spermatozoïde) pour une fécondation réellement effectuée in vivo.

Le seul inconvénient de cette procédure peut être l'absence de programmation à long terme (plus de 24 heures) de la ponction de l'ovocyte. Pour cela un nouveau protocole a été développé faisant appel à de la progestérone. La progestérone semble avoir des effets contreversés sur la LH, pour certains elle provoque la survenue du pic de LH, pour d'autres elle l'inhibe.

En fait il a été observé que l'action de la progestérone sur le pic de LH dépend de la dose utilisée et du moment de son administration. L'expérience, sur seulement un sujet volontaire, de l'administration de progestérone en période périovulatoire à la dose de 50 mg par voie vaginale toutes les 12 heures, a permis de confirmer la disparition totale du pic de LH durant plus de 48 heures. L'administration de la progestérone avait été effectuée alors que le taux d'estradiol avait atteint 150 pg.ml soit quelques heures avant la survenue du pic de LH. On pensait que cette inhibition se fait par contrôle de l'hypophyse. Mais un effet inattendu fut la décroissance progressive du taux d'estradiol. Cette décroissance indiquerait donc que la progestérone n'agit pas uniquement, à cette dose, comme inhibiteur de la LH mais aussi comme inhibiteur de la FSH. L'adjonction de FSH naturelle, 2 à 6 ampoules 1 à 2 heures après l'administration de progestérone et suivant le même rythme toutes les 12 heures s'avérerait nécessaire afin de maintenir la croissance des taux d'estradiol, de parfaire la maturité de l'ovocyte et d'éviter une involution trop précoce des cellules de la granulosa, qui serait néfaste à la qualité de l'ovocyte. Cette injection de FSH pourrait aussi trouver une application dans un cycle spontané sans blocage de la LH par la progression, afin de parfaire la maturité de l'ovocyte dans le cas d'une ponction programmée pour des raisons d'organisation trop précocemment. Ainsi, 2 à 6 ampoules de FSH (150 à 450 UI) seraient injectées alors que le premier doublement du taux de base de LH est observé et dès la réception des résultats sanguins. La ponction ovocytaire survenant 34 à 36 heures après le prélèvement sanguin. Ce protocole serait intéressant chez les patientes agées de moins de 40 ans avec stagnation de la LH.

## Revendications

1. Procédé de fécondation in vitro adapté à être réalisé de façon ambulatoire dans lequel après ponction ovocytaire, on introduit un ou plusieurs de ces ovocytes dans un conteneur renfermant un milieu de culture, sans interposition de l'air ou CO₂, ainsi que des spermatozoïdes, avant l'introduction du conteneur dans la cavité vaginale ou l'utérus en vue de la fécondation du ou des ovocytes et la culture du ou des embryons, les valeurs de l'estradiol (E2) et de la LH étant surveillées par des dosages sanguins, caractérisé en ce que le procédé est réalisé sans stimulation du cycle ni injection de gonadotrophine chorionique avant ponction ovocytaire et la programmation de la ponction ovocytaire est basée sur les valeurs de l'E2 après doublement de la valeur de la LH pour la première fois.

2. Procédé de fécondation selon la revendication 1 caractérisé en ce que les taux sanguins de l'E2 et de la LH sont déterminés 2 fois par jour, une fois entre 8 et 10 heures et une fois entre 15 et 17 heures.

3. Procédé de fécondation selon la revendication 2, caractérisé en ce que, en présence d'une baisse de 30% ou d'une stabilité (c'est-à-dire ± 10% de la valeur antérieure) de l'E2 par rapport à la valeur lors du doublement de la LH pour la première fois, la ponction est programmée 34 à 36 heures après le premier doublement de la valeur de la LH.

4. Procédé selon l'une des revendications 1 ou 2, caractérisé en ce que, en présence d'une augmentation de plus de 20% de l'E2 par rapport à la valeur du premier doublement de la valeur de la LH, la ponction est effectuée 30 à 34 heures après l'augmentation de plus de 20% de l'E2.

5. Procédé selon la revendication 1 ou 2, caractérisé en ce que la ponction ovocytaire est programmée aussitôt que trois dosages de E2 aient révélé une stabilité (c'est-à-dire ± 10% de la valeur antérieure) après le premier doublement de la valeur de la LH.

6. Procédé selon l'une quelconque des revendications précédentes, caractérisé en ce que l'on effectue la maturation de l'ovocyte après son recueil.

7. Procédé selon l'une quelconque des revendications précédentes, caractérisé en ce que l'ovocyte introduit dans le conteneur sans introduction d'air ou de CO₂ est maintenu à une température de 37° environ pendant deux à six heures.

8. Procédé selon l'une quelconque des revendications précédentes, caractérisé en qu'un seul ovocyte est ponctionné et fécondé.

9. Procédé selon l'une des revendications 1 à 8 caractérisé en ce qu'avant l'introduction du conteneur dans l'utérus on le maintient à une température de 37° environ pendant 2 à 6 heures.

10. Kit pour mettre en oeuvre le procédé selon une quelconque des revendications 1 à 10, caractérisé en ce qu'il comprend un dispositif de dosage rapide de la LH, une aiguille de ponction d'ovocyte, une unité de filtration pour préparation de sperme, un conteneur pour fécondation et/ou maturation du ou des ovocytes et culture du ou des embryons avec ses moyens de contention, et des milieux de culture pour le rinçage du follicule, le lavage du sperme et la maturation et la fécondation de l'ovocyte.

11. Kit selon la revendication 10, caractérisé en ce que le ou les conteneurs sont au moins partiellement biodégradables.

12. Kit selon la revendication 10 ou 11, caractérisé en ce qu'il comprend en outre un speculum jetable.

13. Kit selon l'une quelconque des revendications 10 à 12, caractérisé en ce qu'il comporte en outre un cathéter pour transfert du ou des embryons.

14. Kit selon l'une quelconque des revendications 10 à 13, caractérisé en ce qu'il comprend en outre une seringue d'injection de gonadotrophine chorionique des seringues de ponction d'ovocyte, des seringues de rinçage de follicule.

15. Kit selon l'une quelconque des revendications 10 à 14, caractérisé en ce qu'il comprend en outre des boîtes de Petri, des compresses et une poche plastique pour couvrir la sonde.

16. Kit selon l'une quelconque des revendications 10 à 15, caractérisé en ce que tous les éléments du kit sont stérilisés, mis sous emballage hermétique et à usage unique.

## Claims

1. A process for in vitro fertilisation adapted to be implemented in ambulatory manner wherein after ovocyte tapping one or more of said ovocytes is introduced into a container containing a culture medium without the interposition of air or CO₂, as well as spermatozoa, before introduction of the container into the vaginal cavity or the uterus for the purposes of fertilisation of the ovocyte or ovocytes and culture of the embryo or embryos, the values of oestradiol (E2) and LH being monitored by blood analyses, characterised in that the process is implemented without stimulation of the cycle or injection of chorionic gonadotrophin before ovocyte tapping and programming of ovocyte tapping is based on the values of E2 after doubling of the value of LH for the first time.

2. A fertilisation process according to claim 1 characterised in that the blood rates of E2 and LH are determined twice per day, once between 8.00 and 10.00 hours and once between 15.00 and 17.00 hours.

3. A fertilisation process according to claim 2 characterised in that in the presence of a drop of 30% or stability (that is to say ± 10% of the previous value) of E2 with respect to the value upon doubling of LH for the first time, tapping is programmed 34 to 36 hours after the first doubling of the value of LH.

4. A process according to one of claims 1 and 2 characterised in that in the presence of an increase of more than 20% of E2 with respect to the value of the first doubling of the value of LH, tapping is effected 30 to 34 hours after the increase of more than 20% of E2.

5. A process according to claim 1 or claim 2 characterised in that ovocyte tapping is programmed as soon as three E2 analysis operations have revealed a stability (that is to say ± 10% of the previous value) after the first doubling of the value of LH.

6. A process according to any one of the preceding claims characterised in that maturing of the ovocyte is effected after collection thereof.

7. A process according to any one of the preceding claims characterised in that the ovocyte introduced into the container without the introduction of air or CO₂ is maintained at a temperature of 37° approximately for two to six hours.

8. A process according to any one of the preceding claims characterised in that a single ovocyte is tapped and fertilised.

9. A process according to one of claims 1 to 8 characterised in that prior to introduction of the container into the uterus it is maintained at a temperature of 37° approximately for 2 to 6 hours.

10. A kit for carrying out the process according to any one of claims 1 to 10 characterised in that it comprises a device for rapid determination of LH, an ovocyte tapping needle, a filtration unit for sperm preparation, a container for fertilisation and/or maturing of the ovocyte or ovocytes and culture of the embryo or embryos with the containing means thereof, and culture agents for rinsing of the follicle, washing of the sperm and maturing and fertilisation of the ovocyte.

11. A kit according to claim 10 characterised in that the container or containers are at least partially biodegradable.

12. A kit according to claim 10 or claim 11 characterised in that it also comprises a disposable speculum.

13. A kit according to any one of claims 10 to 12 characterised in that it further comprises a catheter for transfer of the embryo or embryos.

14. A kit according to any one of claims 10 to 13 characterised in that it further comprises a chorionic gonadotrophin injection syringe, ovocyte tapping syringes and follicle rinsing syringes.

15. A kit according to any one of claims 10 to 14 characterised in that it further comprises Petri dishes, compresses and a plastics bag for covering the probe.

16. A kit according to any one of claims 10 to 15 characterised in that all the elements of the kit are sterilised, hermetically packaged, and for once-only use.

## Patentansprüche

1. Verfahren zur in vitro-Befruchtung, welches ambulant, ausgeführt werden kann, in welchem nach Eizellenpunktation man eine oder mehrere dieser Eizellen in einen Behälter einführt, der ein Kulturmilieu einschließt, ohne Zwischenfügung von Luft oder CO₂ sowie Spermien vor der Einführung des Inhalts in den vaginalen Hohlraum oder den Uterus im Hinblick auf Befruchrung des oder der Eizellen und der Kultur des oder der Embryonen, wobei die Werte von Östradiol (E2) und von LH durch Dosierung im Blut überwacht werden,
**dadurch gekennzeichnet,**
daß das Verfahren ohne Stimulation des Zyklus noch Injektion von Chorion-Gonandotrophin ausgeführt wird, und zwar vor der Einpunktation, und daß die Programmierung der Eipunktation auf Werten von E2 beruht, nachdem der Wert von LH zum ersten Mal verdoppelt worden ist.

2. Verfahren der Befruchtung nach Anspruch 1,
**dadurch gekennzeichnet**, daß die Anteile von E2 und LH im Blut zweimal pro Tag bestimmt werden, einmal zwischen 8 und 10 Uhr und einmal zwischen 15 und 17 Uhr.

3. Verfahren der Befruchtung nach Anspruch 2,
**dadurch gekennzeichnet**, daß bei einem Absinken von 30 % oder einer Stabilität von E2 (d. h. ± 10 % des vorhergehenden Wertes) im Verhältnis zum Wert bei der Verdoppelung von LH zum ersten Mal, die Punktation auf 34 bis 36 Stunden nach dem ersten Verdoppeln des Wertes von LH programmiert wird.

4. Verfahren nach einem der Ansprüche 1 oder 2, dadurch gekennzeichnet, daß bei einer Vergrößerung um Mehr als 20 % von E2 mit Bezug auf den Wert der ersten Verdoppelung der Wertes von LH die Punktation 30 bis 34 Stunden nach der Vergrößerung um mehr als 20 % von E2 ausgeführt wird.

5. Verfahren nach Anspruch 1 oder 2,
dadurch gekennzeichnet, daß die Eipunkation sogleich programmiert wird, wenn drei Dosierungen von E2 eine Stabilität gezeigt haben (d. h. ± 10 % des vorhergehenden Wertes), und zwar nach dem ersten Verdoppeln des Wertes von LH.

6. Verfahren nach einem der vorhergehenden Ansprüche, dadurch gekennzeichnet, daß man die Reifung des Eis nach seinem Aufnehmen bewirkt.

7. Verfahren nach einem der vorhergehenden Ansprüche, dadurch gekennzeichnet, daß das in dem Behälter ohne Luft oder CO₂ eingeführte Ei bei einer Temperatur von ungefähr 37°C während 2 bis 6 Stunden gehalten wird.

8. Verfahren nach einem der vorhergehenden Ansprüche, dadurch gekennzeichnet, daß nur ein einziges Ei punktiert und befruchtet wird.

9. Verfahren nach einem der Ansprüche 1 bis 8,
dadurch gekennzeichnet, daß nach der Einführung des Behälters in den Uterus man diesen bei einer Temperatur von ungefähr 37°C während 2 bis 6 Stunden hält.

10. Kit zur Durchführung des Verfahrens nach einem der Ansprüche 1 bis 10,
dadurch gekennzeichnet, daß es eine Vorrichtung zum raschen Dosieren von LH, eine Nadel zur Eipunktation, eine Filtrationseinheit zur Vorbereitung von Sperma, einen Behälter zur Befruchtung und /oder Reilung des oder der Eizellen und Kultur des oder der Embryonen mit Haltemitteln und mit Kulturmilieus zur Spülung des Folikels, der Waschung des Spermas und der Reilung und Befruchtung der Eizelle enthält.

11. Kit nach Anspruch 10,
dadurch gekennzeichnet, daß die Hälteeinrichtungen mindestens teilweise bioabbaubar sind.

12. Kit nach Anspruch 10 oder 11,
dadurch gekennzeichnet, daß er im übrigen einen wegwerfbaren Spiegel umfaßt.

13. Kit nach einem der Ansprüche 10 bis 12,
dadurch gekennzeichnet, daß er im übrigen Katheter zur Übertragung des oder der Embryonen umfaßt.

14. Kit nach einem der Ansprüche 10 bis 13,
dadurch gekennzeichnet, daß er im übrigen eine Spritze zur Injektion von Chorion-Gandotrophin, Spritzen zur Punktation der Eizelle sowie Spritzen zur Spülung des Folikels enthält.

15. Kit nach einem der Ansprüche 10 bis 14,
dadurch gekennzeichnet, daß er im übrigen Petrischalen, Kompressen und eine Kunststofftasche zur Abdeckung der Sonde enthält.

16. Kit nach einem der Ansprüche 10 bis 15,
dadurch gekennzeichnet, daß alie Elemente des Kits sterilisiert sind, hermetisch verpackt sind und zum einmaligen Gerbrauch dienen.
